# EUROPEAN PATENT SPECIFICATION

(11) **EP 1 701 722 B1**
(45) Date of publication and mention of the grant of the patent: **14.10.2009**
(21) Application number: 05817697.5
(22) Date of filing: 22.11.2005
(51) Int. Cl.: A61K 31/41, C07D 277/24

(54) **N-HYDROXY-4-{5-[4-(5-ISOPROPYL-2-METHYL-1,3-THIAZOL-4-YL)PHENOXY¨]PENTOXY}BENZAMIDINE 2 METHANSULFONIC ACID SALT**
N-HYDROXY-4-{5-[4-(5-ISOPROPYL-2-METHYL-1,3-THIAZOL-4-YL)PHENOXY]PENTOXY}BENZAMIDIN 2 METHANSULFONSAURESALZ
SEL D'ACIDE N-HYDROXY-4-}5-[4-(5-ISOPROPYL-2-METHYL-1,3-THIAZOL-4-YL)PHENOXY]PENTOXY}BENZAMIDINE 2 METHANESULFONIQUE

(30) Priority: 23.11.2004 KR 20040096390
(43) Date of publication of application: 20.09.2006
(73) Proprietor: Dong Wha Pharmaceutical Co., Ltd., Seoul 100-130 (KR)
(72) Inventor: RYU, Jei, Man, 207-101, Eunhasu Apt., Anyang-si, Gyeonggi-do 431-054 (KR); LEE, Jin, Soo, 104-801, Samsung 1-cha Apt., Yongin-si, Gyeonggi-do 449-762 (KR); SHIN, Dong, Hyuk, Gunpo-si, Gyeonggi-do 435-040 (KR); SEONG, Seung, Kyoo, 105-1308, Daerim Hansup Apt., Anyang-si, Gyeonggi-do 430-702 (KR); CHO, Soon, Ki, 115-604, e-Pyeonhansesang Apt., Anyang-si, Gyeonggi-do 431-754 (KR); JEON, Chan, Seok, Yongin-si, Gyeonggi-do 446-959 (KR); JIN, Young, Goo, 101-204, Dongbu Centreville, Seoul 120-050 (KR); LEE, Ki, Young, Seoul 153-762 (KR); JUNG, Se, Hyun, Anyang-si, Gyeonggi-do 430-822 (KR); CHO, Eun, Hee, Suwon-si, Gyeonggi-do 441-882 (KR); AHN, Seok Hoon 502 Mugungwha Jutaek, Seocho-gu, Seoul 137-071 (KR)
(74) Representative: Teuten, Andrew John
(86) International application number: PCT/KR2005/003934
(87) International publication number: WO 2006/057501

(56) References cited:
- WO-A1-03/007947
- SAX AND LEWIS.: 'Hawle's Condensed Chemical Dictionary 11th Ed.', 1987, VAN NOSTRAND REINHOLD COMPANY. page 752, XP008081812

## Description

### Technical Field

The present invention relates to N-Hydroxy-4-{5-[4-(5-isopropyl-2-methyl-1,3-thiazol-4-yl)phenoxy]pentoxy} benzamidine bis(methanesulfonate), a method of preparing the compound, and a pharmaceutical composition comprising the compound.

### Background Art

The N-Hydroxy-4-{5-[4-(5-isopropyl-2-methyl-1,3-thiazol-4-yl)phenoxy]pentoxy} benzamidine compound has excellent efficacy in the treatment and prevention of osteoporosis (Korean Pat. Laid-open Publication No. 10-2003-0008654), in the treatment of bone fractures (Korean Pat. Application No. 10-2005-0060425), and in the treatment and prevention of allergic diseases (Korean Pat. Application No. 10-2005-0060439).

It is generally known to those skilled in the art that active ingredients used in pharmaceutical compositions must be highly soluble in water or an aqueous solution of a broad range of pH values. However, since the N-Hydroxy-4-{5-[4-(5-isopropyl-2-methyl-1,3-thiazol-4-yl)phenoxy]pentoxy} benzamidine compound has low solubility, its salt forms having high solubility need to be developed to increase bioavailability of the compound.

In this regard, the present inventors conducted an intensive and thorough study to develop a novel salt form of N-Hydroxy-4-{5-[4-(5-isopropyl-2-methyl-1,3-thiazol-4-yl)phenoxy]pentoxy} benzamidine, which is highly soluble and stable. The study resulted in the finding that N-Hydroxy-4-{5-[4-(5-isopropyl-2-methyl-1,3-thiazol-4-yl)phenoxy]pentoxy} benzamidine bis(methanesulfonate) has excellent physicochemical properties (stability, solubility and bioavailability), and that the preparation method of the compound is highly reproducible, thereby leading to the present invention.

### Disclosure of the Invention

It is therefore an object of the present invention to provide N-hydroxy-4-{5-[4-(5-isopropyl-2-methyl-1,3-thiazol-4-yl)phenoxy]pentoxy}benzamidine bis(methanesulfonate).

It is another object of the present invention to provide a method of preparing the N-hydroxy-4-{5-[4-(5-isopropyl-2-methyl-1,3-thiazol-4-yl)phenoxy]pentoxy}benzamidine bis(methanesulfonate).

It is a further object of the present invention to provide a pharmaceutical composition for preventing and treating osteoporosis, bone fractures and allergic inflammatory diseases, comprising the N-hydroxy-4-{5-[4-(5-isopropyl-2-methyl-1,3-thiazol-4-yl)phenoxy]pentoxy}benzamidine bis(methanesulfonate) and a pharmaceutically acceptable carrier.

### Best Mode for Carrying Out the Invention

In one aspect, the present invention relates to a compound represented by the following formula, N-hydroxy-4-{5-[4-(5-isopropyl-2-methyl-1,3-thiazol-4-yl)phenoxy]pentoxy}benzamidine bis(methanesulfonate).

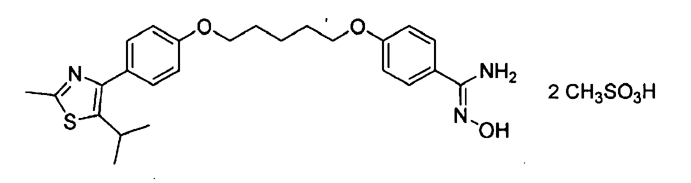

The N-hydroxy-4-{5-[4-(5-isopropyl-2-methyl-1,3-thiazol-4-yl)phenoxy]pentoxy}benzamidine compound and pharmaceutically acceptable salts thereof are disclosed in Korean Pat. Laid-open Publication No. 10-2003-0008654 and International Pat. Publication No. WO03/007947. N-hydroxy-4-{5-[4-(5-isopropyl-2-methyl-1,3-thiazol-4-yl)phenoxy]pentoxy}benzamidine methanesulfonate is mentioned.

The present invention is directed to the bis(methanesulfonate) of the benzamidine compound. The "bis (methanesulfonate) ", as used herein, refers to a compound in which two methanesulfonic acid molecules are bonded to one free base compound to form a salt, and with respect to the present objects, indicates a bis(methanesulfonate) of N-hydroxy-4-{5-[4-(5-isopropyl-2-methyl-1,3-thiazol-4-yl)phenoxy]pentoxy}benzamidine.

The present inventors found that a bis(methanesulfonate), in which two methanesulfonic acid molecules are bonded to the N-hydroxy-4-{5-[4-(5-isopropyl-2-methyl-1,3-thiazol-4-yl)phenoxy]pentoxy}benzamidine compound having low solubility, has higher solubility and exerts remarkably higher bioavailability *in vivo*, than a monomethanesulfonate in which one methanesulfonic acid molecule is bonded to the benzamidine compound.

In detail, the bis(methanesulfonate) of N-hydroxy-4-{5-[4-(5-isopropyl-2-methyl-1,3-thiazol-4-yl)phenoxy]pentoxy}benzamidine according to the present invention exhibited solubility about 8.5-fold higher in distilled water and about 3-fold higher at pH 4.0, than the monomethanesulfonate. Also, when administered to the body, the bis(methanesulfonate) displayed high bioavailability of greater than 46% compared to the monomethanesulfonate of the benzamidine compound.

The bis(methanesulfonate) of N-hydroxy-4-{5-[4-(5-isopropyl-2-methyl-1,3-thiazol-4-yl)phenoxy]pentoxy}benzamidine according to the present invention may be in a crystal or non-crystal form. Preferred is a crystal form of the bis(methanesulfonate) of N-hydroxy-4-{5-[4-(5-isopropyl-2-methyl-1,3-thiazol-4-yl)phenoxy]pentoxy}benzamidine.

In another aspect, the present invention relates to a method of preparing the bis(methanesulfonate) of N-hydroxy-4-{5-[4-(5-isopropyl-2-methyl-1,3-thiazol-4-yl)phenoxy]pentoxy}benzamidine.

In detail, the present invention provides a method of preparing the bis(methanesulfonate) of N-hydroxy-4-{5-[4-(5-isopropyl-2-methyl-1,3-thiazol-4-yl)phenoxy]pentoxy}benzamidine, comprising reacting N-hydroxy-4-{5-[4-(5-isopropyl-2-methyl-1,3-thiazol-4-yl)phenoxy]pentoxy}benzamidine and methanesulfonic acid in an inert solvent.

The methanesulfonic acid used in the present method, which is a salt that has been approved for use in drugs by the US FDA, is a colorless stable liquid that is not hygroscopic and not corrosive. Also, the methanesulfonic acid is not toxic, so it provides a safe environment during production, and it is easy to handle, so it can be readily mass-produced.

The preparation of the bis(methanesulfonate) of N-hydroxy-4-{5-[4-(5-isopropyl-2-methyl-1,3-thiazol-4-yl)phenoxy]pentoxy}benzamidine according to the present invention is reproducible even when methanesulfonic acid is used in an excessive amount. In contrast, if precise equivalents and conditions are not fulfilled, the monomethanesulfonate is not obtained at a certain quantity. Thus, the bis(methanesulfonate) is advantageous because its preparation is reproducible. Since the bis(methanesulfonate), unlike the monomethanesulfonate, is easy to mass-produce due to its reproducibility, it is more beneficial in industrial applications for treating or preventing diseases.

The inert solvent useful in the present method includes ethyl acetate, methanol, ethanol, isopropanol, acetone, acetonitrile, hexane, and isopropyl ether. Of these, ethanol is most preferred.

In the inert solvent, one equivalent of N-hydroxy-4-{5-[4-(5-isopropyl-2-methyl-1,3-thiazol-4-yl)phenoxy]pentoxy}benzamidine is reacted with 2 to 4 equivalents, preferably 2.1 to 2.5 equivalents, of methanesulfonic acid, at -20°C to 40°C, preferably 0°C to 20°C, for 10 min to 5 hrs, preferably 30 min to 2 hrs.

Through the present method, the bis(methanesulfonate) of N-hydroxy-4-{5-[4-(5-isopropyl-2-methyl-1,3-thiazol-4-yl)phenoxy]pentoxy}benzamidine may be produced in high yield of 88% or higher.

In a further aspect, the present invention relates to a pharmaceutical composition for preventing and treating osteoporosis, comprising the bis(methanesulfonate) of N-hydroxy-4-{5-[4-(5-isopropyl-2-methyl-1,3-thiazol-4-yl)phenoxy]pentoxy}benzamidine. Also, the present invention relates to a pharmaceutical composition for treating bone fractures, comprising the bis(methanesulfonate) of N-hydroxy-4-{5-[4-(5-isopropyl-2-methyl-1,3-thiazol-4-yl)phenoxy]pentoxy}benzamidine. Moreover, the present invention relates to a pharmaceutical composition for preventing and treating allergic inflammatory diseases, comprising the bis(methanesulfonate) of N-hydroxy-4-{5-[4-(5-isopropyl-2-methyl-1,3-thiazol-4-yl)phenoxy]pentoxy}benzamidine.

As used herein, the term "osteoporosis", which is also called 'osteopenia', indicates a condition that features the excess loss of inorganic and organic matrix of bone with no structural abnormality in the remaining bone, leading to the bone to be full of tiny holes like a sponge and thus compressible and fragile. The excellent clinical efficacy of the N-hydroxy-4-{5-[4-(5-isopropyl-2-methyl-1,3-thiazol-4-yl)phenoxy]pentoxy}benzamidine compound in the prevention and treatment of osteoporosis is described in detail in Korean Pat. Laid-open Publication No. 10-2003-0008654 and International Pat. Publication No. WO03/007947.

The term "bone fractures", as used herein, which describes a state in which the continuity of bone tissue is disrupted completely or incompletely, includes various physical injuries of the bone, which are classified based on anatomic location (epiphyseal, metaphyseal, diaphyseal and intra-articular, or proximal, middle and distal, etc.), severity of fractures (complete, incomplete, etc.), direction of fractures (transverse, oblique, spiral, longitudinal, etc.), the presence of open wounds (open, closed), the number of fracture fragments (simple or linear, comminuted, segmental, etc.), stability of fractures (stable, unstable), and the degree of displacement of fracture fragments. In rats, the N-hydroxy-4-{5-[4-(5-isopropyl-2-methyl-1,3-thiazol-4-yl)phenoxy]pentoxy}benzamidine compound significantly reduced callus volume compared to a control not treated with the benzamidine compound, significantly enhanced bone mineral content and mechanical strength of callus, significantly reduced the content of connective and soft tissues in the callus tissue, and significantly increased bone tissue density (Korean Pat. Application NO. 10-2005-0060425).

The term "allergic inflammatory diseases", as used herein, refers to non-specific inflammatory diseases caused by a variety of allergens, and includes allergic rhinitis, asthma, allergic conjunctivitis, allergic dermatitis, atopic dermatitis, contact dermatitis, urticaria, anaphylaxis, insect allergy, food allergy, and drug allergy. The preventive and therapeutic efficacy of the N-hydroxy-4-{5-[4-(5-isopropyl-2-methyl-1,3-thiazol-4-yl)phenoxy]pentoxy}benzamidine compound on allergic inflammatory diseases was confirmed in a mouse model of asthma which was induced by chronic exposure to ovalbumin. The benzamidine compound was administered for a period of 18 days, starting on the day of immunization with ovalbumin. 15 days after immunization, experimental animals were challenged with ovalbumin, and sacrificed three days later to investigate lung weight, changes in the cellular profile of bronchoalvelar lavage fluid and peripheral blood samples, and histopathological changes in lung tissue. The oral administration of the benzamidine compound suppressed the increase in lung weight compared to a control administered only with sterile distilled water. The total number of leukocytes and the number of eosinophils significantly increased in asthmatic mice compared to normal mice, but significantly decreased in asthmatic mice administered with the benzamidine compound in a dose-dependent manner compared to asthmatic mice (control group) not administered the benzamidne compound. Also, the number of eosinophils in bronchoalveolar lavage fluid significantly increased in asthmatic mice compared to normal mice, but significantly decreased in asthmatic mice administered with the benzamidine compound in a dose-dependent manner compared to the control group. The asthmatic mice administered with the benzamidine compound exhibited significantly increased alveolar area compared to the control group (Korean Pat. Application NO. 10-2005-0060439).

In addition to the aforementioned component, the present composition may further include one or more pharmaceutically acceptable carriers. The pharmaceutically acceptable carriers may include ordinary excipients, disintegrants, humectants, fillers, thickeners, binders, lubricants, antioxidants, buffering agents, surfactants, dispersing agents, and their combinations of two or more.

The present composition may be administered orally or parenterally. For oral administration, the composition may be formulated into solid forms, for example, tablets, capsules, pills or powders, or into liquid forms, for example, suspensions, syrups or solutions. For pareteral administration (e.g., intravenous, subcutaneous, intraperitoneal, intranasal, etc.), the composition may be formulated into injections, ointments, patches, or the like. These formulations may be suitably made depending on the type of diseases or ingredients according to a proper method known in the art or a method described in the literature: Remington's Pharmaceutical Science (recent version), Mack Publishing Company, Easton PA.

Preferably, oral formulations may be prepared using one or more carbonates, selected from the group consisting of alkali metal carbonate, alkali metal bicarbonate and alkaline earth metal carbonate, and/or one or more disintegrants selected from the group consisting of sodium starch glycolate, calcium carmellose and sodium croscarmellose. This formulation increases the release rate of the bis(methanesulfonate) and remarkably enhances the bioavailability of the bis(methanesulfonate) by suppressing the gelation of the bis(methanesulfonate) by contact with water in the early stage of release. The aforementioned carbonate and/or disintegrant regionally forms a neutral pH or weak alkaline environment in the diffusion layer contacting with water during release of the bis(methanesulfonate), or rapidly disperses the composition, thereby effectively suppressing the gelation caused by hydration in the early stage of release.

The carbonate used in the oral formulations is selected from the group consisting of alkali metal carbonate, such as sodium carbonate, potassium carbonate, or the like; alkali metal bicarbonate, such as sodium bicarbonate, potassium bicarbonate, or the like; and alkaline earth metal carbonate, such as calcium carbonate, magnesium carbonate, or the like. Sodium bicarbonate or calcium carbonate is preferred. The carbonate may be contained in an amount of about 0.4 to 6 parts by weight, preferably 0.5 to 2 parts by weight, based on one part by weight of the bis(methanesulfonate). When the carbonate is used in an amount of less than 0.4 parts by weight, the release rate of the compound is not enhanced. Carbonate of greater than 6 parts by weight generates gas in the gastrointestinal tract and thus causes abdominal swelling.

The disintegrant used in the oral formulations is one or more selected from the group consisting of sodium starch glycolate, calcium carmellose and sodium croscarmellose. Sodium starch glycolate or sodium croscarmellose is preferred. The disintegrants rapidly absorb water and largely swell in the early stage of release to disperse particles of the compound of the above formula, thereby effectively suppressing gelation beginning on the surface of formulations, resulting in increased release rates of the compound. The content of the disintegrant ranges from 0.5 to 5 parts by weight, based on one part by weight of the bis(methanesulfonate) of the above formula. When the disintegrant is used in an amount of less than 0.5 parts by weight, the drug is not evenly dispersed, leading to a decrease in the suppressive effect of the carrier against gelation in the early stage of release, and eventually resulting in no improvement in the release rate of the drug. The disintegrant of greater than 5 parts by weight does not exhibit an enhancing effect on the release rates of the drug any more, and enlarges the volume of the formulations, thereby causing inconvenience upon ingestion of the drug and resulting in decreased patient compliance.

The oral formulation may be prepared by mixing the bis(methanesulfonate) with both the disintegrant and carbonate. The combinational use of the disintegrant and carbonate improves the release properties of the drug relative to single use. In the case of the combinational use of the disintegrant and carbonate, the oral formulation of the present invention preferably contains the disintegrant in an amount of 0.5 to 5 parts by weight and the carbonate in an amount of 0.1 to 6 parts by weight, based on one part by weight of the bis(methanesulfonate). When the disintegrant and carbonate are used in amounts of less than 0.5 and 0.1 parts by weight, respectively, they do not exhibit a suitable inhibitory effect on gelation. When the amounts of disintegrant and carbonate exceed 5 and 6 parts by weight, respectively, satisfactory patient compliance is not achieved.

In addition, the oral formulation may further include an excipient. In order to increase the release rate of the drug by effectively inhibiting gelation and thus rapidly dispersing the drug, the excipient is preferably an inorganic excipient, such as calcium biphosphate, calcium phosphate, heavy magnesium oxide, precipitated calcium carbonate, or magnesium carbonate. More preferred is calcium biphosphate, calcium phosphate, or heavy magnesium oxide. In contrast, organic excipients, such as avicel, mannitol, corn starch and lactose, have no enhancing effect on the release rate of the drug.

The oral formulation may further a pharmaceutically-acceptable ordinary additive. Examples of the additive include binders, lubricants, surfactants, colorants, and taste/smell masking agents. Pharmaceutically-acceptable ordinary binders and lubricants are available. The binders are exemplified by maltose, Arabia gum and hydroxypropylcellulose. The lubricants are exemplified by carnauba wax, light anhydrous silic acid, synthetic aluminum silicate, stearic acid, magnesium stearate, and talc.

In addition to the aforementioned components, the present composition may include a pharmaceutically-acceptable ordinary excipient or adjuvant, and may be formulated into a solid formulation for oral administration, such as tablets, capsules, granules, or fine granules, through an ordinary pharmaceutical method. That is, according to the present invention, the composition may be formulated as granules, and may be supplemented with a lubricant and other pharmaceutically acceptable additives and directly filled into hard capsules in a powder or granule form. Otherwise, the composition may be supplemented with pharmaceutical additives for tabletting and compressed to produce tablets according to a known method.

The dosage may vary according to the patient's weight, age, gender, health state and diet, administration duration, administration routes, excretion rates, severity of the illness, and the like. The bis(methanesulfonate) of N-Hydroxy-4-{5-[4-(5-isopropyl-2-methyl-1,3-thiazol-4-yl)phenoxy]pentoxy} benzamidine contained in the present composition may be administered, for example, in a daily dosage of 1 to 1,000 mg/kg, preferably 10 to 500 mg/kg. The daily dosage may be divided into one to several doses.

The present composition may be used singly or in combination with surgical operation, hormone therapy, drug therapy and biological response regulators in order to prevent and treat osteoporosis, bone fractures and allergic inflammatory diseases.

A better understanding of the present invention may be obtained through the following examples which are set forth to illustrate, but are not to be construed as the limit of the present invention.

### EXAMPLES

The bis(methanesulfonate) of N-Hydroxy-4-{5-[4-(5-isopropyl-2-methyl-1,3-thiazol-4-yl)phenoxy]pentoxy} benzamidine according to the present invention was prepared and evaluated for solubility, stability and bioavailability in the following examples.

### REFERENCE PREPARATION EXAMPLE 1: Preparation of N-Hydroxy-4-{5-[4-(5-isopropyl-2-methyl-1,3-thiazol-4-yl)phenoxy]pentoxy} benzamidine

N-Hydroxy-4-{5-[4-(5-isopropyl-2-methyl-1,3-thiazol-4-yl)phenoxy]pentoxy} benzamidine was prepared according to a method described in the literature: SU Lee, Synthesis and Biological Activity of Natural Products and Designed New Hybrid Compounds for the treatment of LTB4 Related Disease, the doctoral thesis, the Graduate School, Busan National University, 1999 August).

### EXAMPLE 1: Preparation of the bis(methanesulfonate) of N-Hydroxy-4-{5-[4-(5-isopropyl-2-methyl-1,3-thiazol-4-yl)phenoxy]pentoxy} benzamidine

150 g (0.33 mol) of N-Hydroxy-4-{5-[4-(5-isopropyl-2-methyl-1,3-thiazol-4-yl)phenoxy]pentoxy} benzamidine was dissolved in 1.1 L of ethanol, and was mixed with 47 mL (2.2 equivalents) of methanesulfonic acid with agitation at room temperature for 1 hr. The solution was then mixed with 3 L of acetone and 1.1 L of hexane with agitation for 1 hr. The thus produced solid was recovered by filtration, washed with acetone, and dried under vacuum. As a result, 188 g (yield: 88%) of N-Hydroxy-4-{5-[4-(5-isopropyl-2-methyl-1,3-thiazol-4-yl)phenoxy]pentoxy} benzamidine bis(methanesulfonate) was obtained as a white solid.

The obtained N-Hydroxy-4-{5-[4-(5-isopropyl-2-methyl-1,3-thiazol-4-yl)phenoxy]pentoxy} benzamidine bis(methanesulfonate) was analyzed for methanesulfonic acid content and melting point, and the results are given in Table 1, below.

**TABLE 1**

| | Methanesulfonic acid content |
|---|---|
| Theoretical value | 29.76% |
| Measured value | 30.02% |

| | |
|---|---|
| Melting point: 156.4°C | |

### COMPARATIVE EXAMPLE 1: Preparation of the monomethanesulfonate N-Hydroxy-4-{5-[4-(5-isopropyl-2-methyl-1,3-thiazol-4-yl)phenoxy]pentoxy} benzamidine

10 g (0.022 mol) of N-Hydroxy-4-{5-[4-(5-isopropyl-2-methyl-1,3-thiazol-4-yl)phenoxy]pentoxy} benzamidine was dissolved in 50 mL of ethanol, and was mixed with 1.43 mL (1 equivalent) of methanesulfonic acid, dissolved in 22 mL of ethanol, with agitation at room temperature for 1 hr. The solvent was then removed under pressure. The reaction mixture was dissolved in 20 ml of ethanol, and was then mixed with 40 mL of acetone and 100 mL of hexane with agitation for 4 hrs. The thus produced solid was recovered by filtration, washed with acetone, and dried under vacuum. As a result, 9.8 g (yield: 81%) of N-Hydroxy-4-{5-[4-(5-isopropyl-2-methyl-1,3-thiazol-4-yl)phenoxy]pentoxy} benzamidine monomethanesulfonate was obtained as a white solid.

The obtained N-Hydroxy-4-{5-[4-(5-isopropyl-2-methyl-1,3-thiazol-4-yl)phenoxy]pentoxy} benzamidine monomethanesulfonate was analyzed for methanesulfonic acid content and melting point, and the results are given in Table 2, below.

**TABLE 2**

| | Methanesulfonic acid content |
|---|---|
| Theoretical value | 17.48% |
| Measured value | 17.68% |

| | |
|---|---|
| Melting point: 110.2°C | |

### EXAMPLE 2: Evaluation of solubility of the bis(methanesulfonate) of N-Hydroxy-4-{5-[4-(5-isopropyl-2-methyl-1,3-thiazol-4-yl)phenoxy]pentoxy} benzamidine

The bis(methanesulfonate), prepared in Example 1, and the monomethanesulfonate, prepared in Comparative Example 1, of N-Hydroxy-4-{5-[4-(5-isopropyl-2-methyl-1,3-thiazol-4-yl)phenoxy]pentoxy} benzamidine, and the N-Hydroxy-4-{5-[4-(5-isopropyl-2-methyl-1,3-thiazol-4-yl)phenoxy]pentoxy} benzamidine, prepared in Reference Preparation Example 1, were examined for solubility (µg/mL) in various solvents at room temperature. The results are given in Table 3, below.

**TABLE 3**

| Solvent | Free base | Used salt | |
|---|---|---|---|
| | | monomethanesulfonate | bis(methanesulfonate) |
| Distilled water | 3.48 | 414.34 | 3,535.33 |
| pH 1.2 | 950.87 | 1,092.98 | 1,686.71 |
| pH 4.0 | - | 0.99 | 3.00 |

As shown in Table 3, the bis(methanesulfonate) of N-Hydroxy-4-{5-[4-(5-isopropyl-2-methyl-1,3-thiazol-4-yl)phenoxy]pentoxy} benzamidine exhibited higher solubility than N-Hydroxy-4-{5-[4-(5-isopropyl-2-methyl-1,3-thiazol-4-yl)phenoxy]pentoxy} benzamidine and its monomethanesulfonate). The bis(methanesulfonate) exhibited solubility about 3-fold higher at pH 4.0 and about 9-fold higher in distilled water.

### EXAMPLE 3: Evaluation of stability of the bis(methanesulfonate) of N-Hydroxy-4-{5-[4-(5-isopropyl-2-methyl-1,3-thiazol-4-yl)phenoxy]pentoxy} benzamidine

The bis(methanesulfonate) of N-Hydroxy-4-{5-[4-(5-isopropyl-2-methyl-1,3-thiazol-4-yl)phenoxy]pentoxy} benzamidine, prepared in Example 1, was placed into a transparent glass vial, and stored at a cap-opened state under accelerated conditions (40±2°C/75±5% RH) for a period of two weeks. Thereafter, the sample was analyzed by HPLC (Waters Module 1). The analysis was performed using a column packed with an octadecyl-silylated silica gel (Shiseido CAPCELL PAK C18, UG 120, Particle size 5 µm) under conditions: UV detection: 256 nm, injection volume: 10 µl, mobile-phase flow rate: 1.5 mL/min. The amount of the compound was calculated as an area percentage. The results are given in Table 4, below.

**TABLE 4**

| | Content (%) |
|---|---|
| Early stage | 99.85 |
| After 2 days | 99.86 |
| After 1 week | 99.87 |
| After 2 weeks | 99.86 |

As shown in Table 4, the accelerated test at 40°C resulted in no change in content of the N-Hydroxy-4-{5-[4-(5-isopropyl-2-methyl-1,3-thiazol-4-yl)phenoxy]pentoxy} benzamidine bis(methanesulfonate) in distilled water. Also, the bis(methanesulfonate) was found to have high chemical stability at high temperature.

### EXAMPLE 4: Pharmacokinetic evaluation of the bis(methanesulfonate) of N-Hydroxy-4-{5-[4-(5-isopropyl-2-methyl-1,3-thiazol-4-yl)phenoxy]pentoxy} benzamidine

The bis(methanesulfonate), prepared in Example 1, and the monomethanesulfonate, prepared in Comparative Example 1, of N-Hydroxy-4-{5-[4-(5-isopropyl-2-methyl-1,3-thiazol-4-yl)phenoxy]pentoxy} benzamidine, and the N-Hydroxy-4-{5-[4-(5-isopropyl-2-methyl-1,3-thiazol-4-yl)phenoxy]pentoxy} benzamidine, prepared in Reference Preparation Example 1, were individually administered to SD rats in a dosage of 50 mg/kg. At given time points (0, 0.5, 1, 1.5, 2, 3, 5 and 8 hrs), rats were mildly anesthetized with diethyl ether, and blood samples were collected from the orbital venous plexus and stored at -20°C until concentration analysis. The plasma samples were mixed with an equal volume of an internal standard substance solution (prepared by dissolving betamethasone in acetonitrile to give a final concentration of 30 µg/ml) with agitation for 1 min, and was centrifuged at 12,000 rpm for 10 min. Active components of the plasma samples were analyzed by HPLC (Model: Waters Module 1). From the obtained data, pharmacokinetic parameters (maximum blood concentration (Cₘₐₓ) and area under the blood concentration-time curve (AUC) were calculated by noncompartment analysis using the WinNonlin program (Version 1.0, Scientific Consulting Inc., USA). The results are given in Table 5, below.

**TABLE 5**

| | Free base | Used salt | |
|---|---|---|---|
| | | monomethanesulfonate | bis(methanesulfonate) |
| Dosage | 50 mg/kg | 50 mg/kg | 50 mg/kg |
| Rat no. | 4 | 4 | 4 |
| Cmax (µg/ml) | 1.09±0.17 | 1.40±0.11 | 1.67±0.32^{*} |
| AUC (µg/ml) | 5.31±0.49 | 7.01±0.60^{*} | 10.28±0.80^{*,#} |

| | | | |
|---|---|---|---|
| ^{*}P<0.05 (relative to free base) ^{#}P<0.05 (relative to monomethanesulfonate) | | | |

As shown in Table 5, in distilled water, the N-Hydroxy-4-{5-[4-(5-isopropyl-2-methyl-1,3-thiazol-4-yl)phenoxy]pentoxy} benzamidine bis(methanesulfonate) exhibited a bioavailability of 46% or higher relative to the monomethanesulfonate.

### Industrial Applicability

As described hereinbefore, the bis(methanesulfonate) of N-Hydroxy-4-{5-[4-(5-isopropyl-2-methyl-1,3-thiazol-4-yl)phenoxy]pentoxy} benzamidine according to the present invention has excellent solubility relative to the monomethanesulfonate of the benzamidine compound, and thus has improved bioavailability. **Thus, the** bis(methanesulfonate) is effective in osteoporosis, bone fractures and allergic inflammatory diseases even at low concentrations and is thus applicable for preventing or treating these diseases.

## Claims

1. N-Hydroxy-4-{5-[4-(5-isopropyl-2-methyl-1,3-thiazol-4-yl)phenoxy]pentoxy} benzamidine bis(methanesulfonate).

2. A method of preparing N-Hydroxy-4-{5-[4-(5-isopropyl-2-methyl-1,3-thiazol-4-yl) phenoxy]pentoxy} benzamidine bis(methanesulfonate), comprising reacting N-Hydroxy-4-{5-[4-(5-isopropyl-2-methyl-1,3-thiazol-4-yl)phenoxy]pentoxy} benzamidine and methanesulfonic acid in an inert solvent.

3. A pharmaceutical composition for preventing and treating osteoporosis, comprising N-hydroxy-4-{5-[4-(5-isopropyl-2-methyl-1,3-thiazol-4-yl)phenoxy]pentoxy}benzamidine bis(methanesulfonate) and a pharmaceutically acceptable carrier.

4. A pharmaceutical composition for treating bone fractures, comprising N-hydroxy-4-{5-[4-(5-isopropyl-2-methyl-1,3-thiazol-4-yl)phenoxy]pentoxy}benzamidine bis(methanesulfonate) and a pharmaceutically acceptable carrier.

5. A pharmaceutical composition for preventing and treating allergic inflammatory diseases, comprising N-hydroxy-4-{5-[4-(5-isopropyl-2-methyl-1,3-thiazol-4-yl)phenoxy]pentoxy}benzamidine bis(methanesulfonate) and a pharmaceutically acceptable carrier.

6. An oral formulation comprising N-hydroxy-4-{5-[4-(5-isopropyl-2-methyl-1,3-thiazol-4-yl)phenoxy]pentoxy}benzamidine bis(methanesulfonate), along with (a) a carbonate selected from the group consisting of alkali metal carbonate, alkali metal bicarbonate and alkaline earth metal carbonate; (b) a disintegrant selected from the group consisting of sodium starch glycolate, calcium carmellose and sodium croscarmellose; or a combination of (a) and (b).

7. The oral formulation as set forth in claim 6, further comprising an inorganic excipient.

8. The oral formulation as set forth in claim 7, wherein the inorganic excipient is calcium biphosphate, calcium phosphate, heavy magnesium oxide, precipitated calcium carbonate, magnesium carbonate, or a mixture thereof.

9. The oral formulation as set forth in any one of claims 6 to 8, wherein the carbonate is sodium bicarbonate or calcium carbonate, and the disintegrant is sodium starch glycolate or sodium croscarmellose.

10. N-Hydroxy-4-{5-[4-(5-isopropyl-2-methyl-1,3-thiazol-4-yl)phenoxy]pentoxy} benzamidine bis(methanesulfonate) for use as a medicament.

## Patentansprüche

1. N-Hydroxy-4-{5-[4-(5-isopropyl-2-methyl-1,3-thiazol-4-yl)phenoxy]pentoxy}benzamidin-bis (methansulfonat) .

2. Verfahren zur Herstellung von N-Hydroxy-4-{5-[4-(5-isopropyl-2-methyl-1,3-thiazol-4-yl)phenoxy]-pentoxy}benzamidin-bis(methansulfonat), bei dem man N-Hydroxy-4-{5-[4-(5-isopropyl-2-methyl-1,3-thiazol-4-yl)phenoxy]pentoxy}benzamidin in einem inerten Lösungsmittel mit Methansulfonsäure umsetzt.

3. Pharmazeutische Zusammensetzung zur Prävention und Behandlung von Osteoporose, die N-Hydroxy-4-{5-[4-(5-isopropyl-2-methyl-1,3-thiazol-4-yl)phenoxy]-pentoxy}benzamidin-bis(methansulfonat) und einen pharmazeutisch unbedenklichen Träger enthält.

4. Pharmazeutische Zusammensetzung zur Behandlung von Knochenbrüchen, die N-Hydroxy-4-{5-[4-(5-isopropyl-2-methyl-1,3-thiazol-4-yl)phenoxy]pentoxy}benzamidin-bis(methansulfonat) und einen pharmazeutisch unbedenklichen Träger enthält.

5. Pharmazeutische Zusammensetzung zur Prävention und Behandlung allergischer entzündlicher Krankheiten, d i e N-Hydroxy-4-{5-[4-(5-isopropyl-2-methyl-1,3-thiazol-4-yl)phenoxy]pentoxy}benzamidin-bis(methansulfonat) und einen pharmazeutisch unbedenklichen Träger enthält.

6. Orale Formulierung, die N-Hydroxy-4-{5-[4-(5-isopropyl-2-methyl-1,3-thiazol-4-yl)phenoxy]-pentoxy}benzamidin-bis(methansulfonat) zusammen mit (a) einem aus der aus Alkalicarbonaten, Alkalihydrogencarbonaten und Erdalkalicarbonaten bestehenden Gruppe ausgewählten Carbonat; (b) einem aus der aus Natriumstärkeglykolat, Calciumcarmellose und Natriumcroscarmellose bestehenden Gruppe ausgewählten Sprengmittel; oder einer Kombination von (a) und (b) enthält.

7. Orale Formulierung nach Anspruch 6, die weiterhin einen anorganischen Exzipienten enthält.

8. Orale Formulierung nach Anspruch 7, wobei es sich bei dem anorganischen Exzipienten um Calciumhydrogenphosphat, Calciumphosphat, schweres Magnesiumoxid, gefälltes Calciumcarbonat, Magnesiumcarbonat oder eine Mischung davon handelt.

9. Orale Formulierung nach einem der Ansprüche 6 bis 8, wobei es sich bei dem Carbonat um Natriumhydrogencarbonat oder Calciumcarbonat und bei dem Sprengmittel um Natriumstärkeglykolat oder Natriumcroscarmellose handelt.

10. N-Hydroxy-4-{5-[4-(5-isopropyl-2-methyl-1,3-thiazol-4-yl)phenoxy]pentoxy}benzamidin-bis(methansulfonat) zur Verwendung als Medikament.

## Revendications

1. Bis(méthanesulfonate) de N-hydroxy-4-{5-[4-(5-isopropyl-2-méthyl-1,3-thiazol-4yl)phénoxy]-pentoxy}benzamidine.

2. Méthode de préparation de bis(méthanesulfonate) de N-hydroxy-4-{5-[4-(5-isopropyl-2-méthyl-1,3-thiazol-4-yl)phénoxy]pentoxy} benzamidine comprenant la réaction de N-hydroxy-4-{5-[4-(5-isopropyl-2-méthyl-1,3-thiazol-4-yl)phénoxy]-pentoxy}benzamidine et d'acide méthanesulfonique dans un solvant inerte.

3. Composition pharmaceutique destinée à la prévention et au traitement de l'ostéoporose, comprenant le bis(méthanesulfonate) de N-hydroxy-4-{5-[4-(5-isopropyl-2-méthyl-1,3-thiazol-4-yl)-phénoxy]pentoxy}benzamidine **et un support** pharmaceutiquement acceptable.

4. Composition pharmaceutique destinée au traitement d e **fractures d'os,** comp**renant** le bis(méthanesulfonate) de N-hydroxy-4-{5-[4-(5-isopropyl-2-méthyl-1,3-thiazol-4-yl)phénoxy]-pentoxy}benzamidine **et un support** pharmaceutiquement acceptable.

5. Composition pharmaceutique destinée à la prévention et au traitement de maladies inflammatoires allergiques, comprenant le bis(méthanesulfonate) de N-hydroxy-4-{5-[4-(5-isopropyl-2-méthyl-1,3-thiazol-4-yl)phénoxy]-pentoxy}benzamidine **et un support** pharmaceutiquement acceptable.

6. **Formulation orale comprenant le** bis(méthanesulfonate) de N-hydroxy-4-{5-[4-(5-isopropyl-2-méthyl-1,3-thiazol-4-yl)phénoxy]-pentoxy}benzamidine, conjointement avec (a) un carbonate choisi dans le groupe constitué de carbonate de métal alcalin, de bicarbonate de métal alcalin et de carbonate de métal alcalino-terreux ; (b) un délitant choisi dans le groupe constitué de glycolate d'amidon sodique, de carmellose calcique et de croscarmellose sodique ; ou une combinaison de (a) et (b).

7. Formulation orale selon la revendication 6, comprenant en outre un excipient minéral.

8. Formulation orale selon la revendication 7, **caractérisée en ce que** l'excipient minéral est le biphosphate de calcium, le phosphate de calcium, l'oxyde de magnésium lourd, le carbonate de calcium précipité, le carbonate de magnésium, ou un mélange de ceux-ci.

9. Formulation orale selon l'une quelconque des revendications 6 à 8, **caractérisée en ce que** le carbonate est le bicarbonate de sodium ou le carbonate de calcium, et le délitant est le glycolate d'amidon sodique ou la croscarmellose sodique.

10. Bis(méthanesulfonate) de N-hydroxy-4-{5-[4-(5-isopropyl-2-méthyl-1,3-thiazol-4-yl)phénoxy]-pentoxy}benzamidine destiné à être utilisé comme médicament.
